(19) Europäisches Patentamt
European Patent Office
Office européen des brevets

(11) Veröffentlichungsnummer : **0 548 018 A1**

# (12) EUROPÄISCHE PATENTANMELDUNG

(21) Anmeldenummer : **92810972.7**

(51) Int. Cl.$^5$ : **C07D 417/12**, A61K 31/425

(22) Anmeldetag : **09.12.92**

(30) Priorität : **18.12.91 CH 3751/91**

(43) Veröffentlichungstag der Anmeldung :
**23.06.93 Patentblatt 93/25**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Missbach, Martin, Dr.**
**Cristalinweg 4**
**CH-4310 Rheinfelden (CH)**

## (54) Neue Imidazole.

(57) Neue Imidazole der Formel I

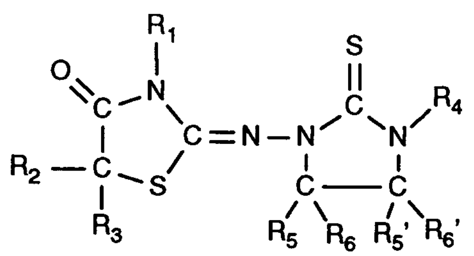

(I)

worin $R_1$ und $R_4$ Niederalkyl, Niederalk-2-en-1-yl oder auch Niederalk-2-in-1-yl bedeuten, $R_2$ und $R_3$ unabhängig voneinander Wasserstoff oder Niederalkyl bedeuten oder gemeinsam für Niederalkyliden stehen, und $R_5$ und $R_6$ jeweils für Wasserstoff oder Niederalkyl stehen oder gemeinsam Oxo darstehen, und $R_5$' und $R_6$' die gleiche Bedeutung von $R_6$ und $R_6$ haben, mit der Massgabe, dass mindestens einer der Substituentenpaare $R_5$ und $R_6$ oder $R_5$' und $R_6$' gemeinsam für Oxo stehen und ihre Salze, Verfahren zur Herstellung der genannten Verbindungen, diese enthaltende pharmazeutische Präparate und ihre Verwendung als Arzneimittelwirkstoffe.

EP 0 548 018 A1

Jouve, 18, rue Saint-Denis, 75001 PARIS

Die Erfindung betrifft neue Imidazole der Formel I

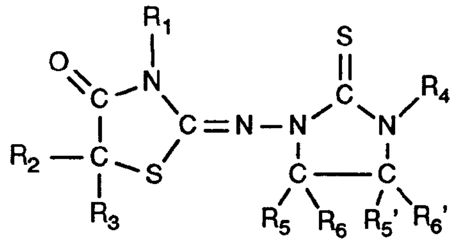

(I)

worin $R_1$ und $R_4$ Niederalkyl, Niederalk-2-en-1-yl oder auch Niederalk-2-in-1-yl bedeuten, $R_2$ und $R_3$ unabhängig voneinander Wasserstoff oder Niederalkyl bedeuten oder gemeinsam für Niederalkyliden stehen, und $R_5$ und $R_6$ jeweils für Wasserstoff oder Niederalkyl stehen oder gemeinsam Oxo darstellen, und $R_6$' und $R_6$' die gleiche Bedeutung von $R_5$ und $R_6$ haben, mit der Massgabe, dass mindestens einer der Substituentenpaare $R_6$ und $R_6$ oder $R_6$' und $R_6$' gemeinsam für Oxo stehen und ihre Salze, Verfahren zur Herstellung der genannten Verbindungen; diese enthaltende pharmazeutische Präparate und ihre Verwendung als Arzneimittelwirkstoffe.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalk-2-en-1-yl ist beispielsweise $C_3$-$C_5$-Alk-2-en-1-yl, wie insbesondere Allyl oder Methallyl.

Niederalk-2-in-1-yl ist beispielsweise $C_3$-$C_5$-Alk-2-in-1-yl, wie insbesondere Prop-2-in-1-yl oder auch But-2-in-1-yl.

Niederalkyl ist beispielsweise $C_1$-$C_4$-Alkyl, wie Methyl, Ethyl, Propyl oder Butyl.

Niederalkyliden ist beispielsweise $C_1$-$C_4$-Alkyliden, wie insbesondere Methylen.

Pharmazeutisch verwendbare Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkan carbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate. Salze von Verbindungen der Formel I sind beispielsweise deren Säureadditionssalze, beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Die Verbindungen der Formel I und ihre pharmazeutisch verwendbaren Salze weisen wertvolle pharmakologische Eigenschaften auf. Insbesondere weisen sie ausgeprägte antiarthritische Eigenschaften auf. Diese können *in vivo* beispielsweise am Modell der Adjuvans-Arthritis der Ratte nach I. Wiesenberg et al. Clin. Exp. Immunol. 78, 245 (1989) in Dosierungen ab etwa 0,1 bis 0,3 mg/kg p.o. oder i.p. nachgewiesen werden.

Die Verbindungen der Formel I und ihre pharmazeutisch verwendbaren Salze können deshalb zur Behandlung von Erkrankungen des rheumatoiden Formenkreises eingesetzt werden. Zu diesen gehören insbesondere die rheumatoide Arthritis, die juvenile Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie Verbindungen der Formel I, worin $R_1$ $C_3$-$C_5$-Alk-2-en-1-yl oder $C_3$-$C_5$-Alk-2-in-1-yl und $R_4$ $C_1$-$C_4$-Alkyl, $C_3$-$C_5$-Alk-2-en-1-yl, oder $C_3$-$C_5$-Alk-2-in-1-yl bedeutet, $R_2$ und $R_3$ unabhängig voneinander Wasserstoff oder $C_1$-$C_4$-Alkyl bedeuten oder gemeinsam $C_1$-$C_4$-Alkyliden darstellen und $R_6$ und $R_6$ bzw. $R_6$' und $R_6$' jeweils unabhängig voneinander Wasserstoff oder $C_1$-$C_4$-Alkyl oder gemeinsam Oxo darstellen, mit der Massgabe, dass mindestens einer der Substituentenpaare $R_6$ und $R_6$ oder $R_6$' und $R_6$' gemeinsam für Oxo stehen, und ihre Salze, insbesondere ihre pharmazeutisch verwendbaren Salze.

Die Erfindung betrifft vor allem Verbindungen der Formel I, worin der Rest $R_1$ $C_3$-$C_5$-Alk-2-en-1-yl, wie Allyl oder Methallyl oder $C_3$-$C_5$-Alk-2-in-1-yl, wie Prop-2-in-2-yl, und $R_4$ $C_1$-$C_4$-Alkyl, wie Methyl, oder $C_3$-$C_5$-Alk-2-en-1-yl, wie Allyl, oder Methallyl, bedeutet, $R_2$ und $R_3$ beide Wasserstoff oder gleiche $C_1$-$C_4$-Alkylgruppen, wie

Methyl, bedeuten oder gemeinsam $C_1$-$C_4$-Alkyliden, wie Methylen darstellen und $R_5$ und $R_6$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_6$' und $R_6$' gemeinsam für Oxo stehen, und ihre Salze, insbesondere ihre pharmazeutisch verwendbare Salze.

Die Erfindung betrifft insbesondere Verbindungen der Formel I, worin $R_1$ Allyl oder Methallyl, $R_2$ und $R_3$ beide Wasserstoff oder Methyl bedeuten, $R_4$ für Methyl, Allyl oder Methallyl stehen und $R_6$ und $R_6$ bzw. $R_6$' und $R_6$' jeweils Wasserstoff oder gemeinsam Oxo darstellen, mit der Massgabe, dass mindestens einer der Substituentenpaare $R_6$ und $R_6$ oder $R_6$' und $R_6$' gemeinsam für Oxo stehen, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Das Verfahren a) zur Herstellung der erfindungsgemäss bereitgestellten Verbindungen ist dadurch gekennzeichnet, dass man einen Verbindung der Formel II

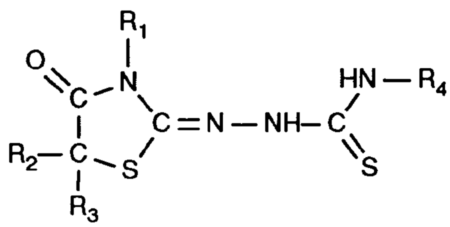

(II)

mit einer entsprechenden Verbindung der Formel III

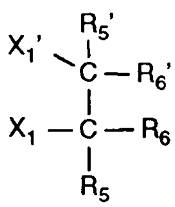

(III)

worin $X_1$ und $X_1$' jeweils ein reaktionsfähiges verestertes Hydroxy bedeuten und $R_5$, $R_6$, $R_5$' und $R_6$' die oben angegebenen Bedeutungen haben, in Gegenwart eines basischen Kondensationsmittel kondensiert.

Die Kondensation erfolgt in Gegenwart eines basischen Kondensationsmittels, wie einer tertiären organischen Base, wie eines Triniederalkylamins, von Hünig'scher Base oder einer organischen Stickstoffbase, wie Pyridin oder Chinolin, und/oder unter Kühlen, vorteilhaft im Temperaturbereich von etwa 25 bis -70°C, beispielsweise von 0 bis -60°C, insbesondere von -40 bis -60°C.

Die Verbindungen der Formel II und auf an sich bekannten Methoden beruhende Verfahren zu Ihrer Herstellung sind bekannt und beispielsweise in GB-1,325,061, US-4,697,020, DE-2,405,395 oder DE 2,632,745 als Zwischenprodukte für die Herstellung von tumorhemmenden Arzneimittelwirkstoffen beschrieben.

In Ausgangsstoffen der Formel III kommen als abspaltbare Reste $X_1$ bzw. $X_1$' beispielsweise reaktionsfähig veresterte Hydroxygruppen, insbesondere Halogen, wie Chlor oder Brom, in Betracht. Verbindungen der Formel III sind bekannt und beispielsweise Oxalylchlorid oder auch Halogenacetylhalogenide, wie z.B. Bromacetylchlorid oder auch -bromid.

Die Kondensation von Verbindungen der Formel II mit Verbindungen der Formel III erfolgt in üblicher Weise, vorteilhaft in einem inerten Lösungsmittel, wie z.B. eines aliphatischen Halogen-Kohlenwasserstoffes, wie in Dichlormethan, oder eines aliphatischen oder cycloaliphatischen Ethers, z.B. in Tetrahydrofuran oder auch Dioxan. In einer bevorzugten Ausführungsform der Verfahrensvariante a) setzt man beispielsweise bei einer Temperatur von -40 bis -60°C eine Verbindung der Formel II in Gegenwart von Triethylamin oder Pyridin und in Dichlormethan oder Tetrahydrofuran als Lösungsmittel mit einer Verbindung der Formel III, z.B. Oxalylchlorid oder einem Halogenacetylhalogenid, wie z.B. Bromacetylchlorid oder -bromid um.

Gemäss einer weiteren Verfahrensvariante b) können Verbindungen der Formel I hergestellt werden, indem man in Verbindungen der Formel IV

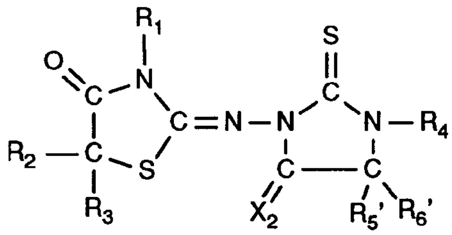

(IV)

worin $X_2$ eine funktionell abgewandelte Oxogruppe darstellt und $R_1$, $R_2$, $R_3$, $R_4$, $R_5$' und $R_5$' die angegebenen Bedeutungen haben, oder in entsprechenden Salzen davon die Gruppe $X_2$ in Oxo überführt.

In Ausgangsstoffen der Formel IV gemäss der Verfahrensvariante b) bedeutet funktionell abgewandeltes Oxo beispielsweise Imino oder Hydroxy.

Ausgangsstoffe der Formel IV werden beispielsweise durch Umsetzung der entsprechenden Verbindungen der Formel II mit Cyanameisensäure oder einem ihrer reaktiven Derivate, Cyanformylchlorid, hergestellt.

Nach einer weiteren Verfahrensvariante c) können Verbindungen der Formel I hergestellt werden, indem man Verbindungen der Formel V

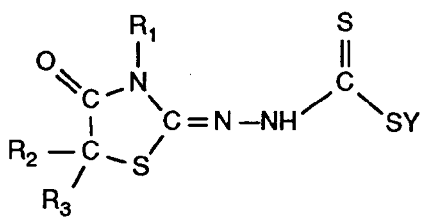

(V)

worin $R_1$, $R_2$, $R_3$ die oben angegebenen Bedeutungen haben und Y Niederalkyl bedeutet, mit Verbindungen der Formeln VI

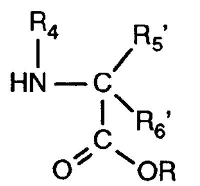

(VI)

worin $R_4$, $R_5$' und $R_5$' die oben angegebenen Bedeutungen haben und R Wasserstoff oder Niederalkyl bedeutet, umsetzt. Der Umsatz einer Verbindung der Formel V mit einer Verbindung der Formel VI erfolgt in einem inerten Lösungsmittel, wie Niederalkanol, beispielsweise Ethanol und gegebenenfalls in Gegenwart eines basischen Kondensationsmittels, wie einer tertiären organischen Base, wie eines Triniederalkylamins, von Hünig'scher Base oder einer organischen Stickstoffbase, wie Pyridin oder Chinolin.

Ausgangsverbindungen der Formel V sind neu und können aus der entsprechenden Hydrazonverbindung der Formel VII

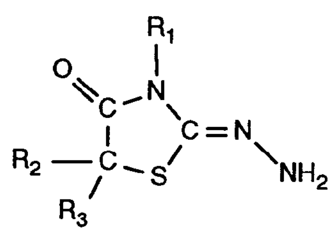

(VII)

durch Umsatz mit Schwefelkohlenstoff, $CS_2$ und anschliessender Reaktion mit einem Niederalkyljodid erhalten werden. Der Umsatz einer Verbindung der Formel VII mit Schwefelkohlenstoff erfolgt in Gegenwart einer tertiären organischen Base, wie eines Triniederalkylamins, von Hünig'scher Base oder einer organischen Stickstoffbase, wie z.B. Pyridin oder Chinolin. Der nachfolgende Umsatz mit einem Niederalkyljodid in situ erfolgt

unter Kühlen des erhaltenen Reaktionsgemisches auf eine Temperatur zwischen -10° und +10°C, vorzugsweise auf eine Temperatur 0° bis +5°C.

Verbindungen der Formel I können auch nach einer weiteren Verfahrensvariante d) hergestellt werden, in denen $R_5$ und $R_6$ gemeinsam Oxo darstellen und $R_5$' und $R_6$' jeweils Wasserstoff bedeuten oder $R_6$' und $R_6$' gemeinsam Oxo dastellen und $R_6$ und $R_6$ jeweils für Wassertoff stehen , indem man eine Verbindung der Formel VIII

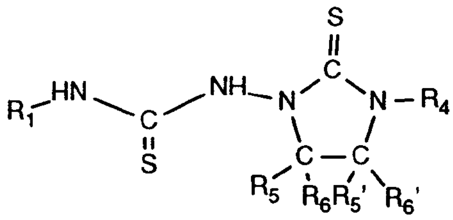

(VIII)

worin $R_1$, $R_4$, $R_6$, $R_6$, $R_5$ ' und $R_6$' die oben angegebenen Bedeutungen haben, mit einer Verbindung der Formel IX

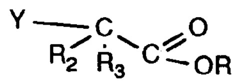

(IX)

worin $R_2$ und $R_3$ die oben angegebenen Bedeutungen haben, Y ein reaktionsfähiges verestertes Hydroxy ist und R Wasserstoff oder Niederalkyl bedeutet, umsetzt. Der Umsatz einer Verbindung der Formel VIII mit einer Verbindung Formel IX erfolgt in einem inerten Lösungsmittel, wie Niederalkanol, beispielsweise Ethanol und gegebenenfalls in Gegenwart eines basischen Kondensationsmittels, wie einer tertiären organischen Base, wie eines Triniederalkylamins, beispielsweise Triethylamins, Hünig'scher Base oder einer organischen Stickstoffbase, wie Pyridin, Dimethylaminopyridin oder Chinolin. Der Umsatz erfolgt vorteilhaft im Temperaturbereich von etwa 0 - 80°C, beispielsweise von 20 - 80°C, insbesondere von 50 - 80°C

Ausgangstoffe der Formel VIII werden erhalten, indem man Verbindungen der Formel X

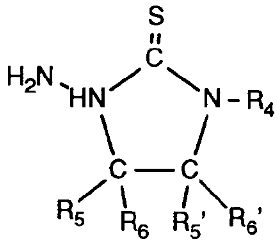

(X)

worin $R_4$ $R_6$, $R_6$, $R_6$' und $R_6$' die unter der Formel VIII angegenen Bedeutungen hat, mit einer Isothiocyanatverbindung der Formel XI

$R_1$-NCS (XI)

umsetzt.

Die Kondensation der Verbindungen der Formel X mit Verbindungen der Formel XI erfogt in üblicher Weise in einem inerten Lösungsmittel, wie Niederalkanol, beispielsweise Ethanol im Temperaturbereich von 0-70°C, beispielsweise 20-70°C, vorteilhafterweise 50-70°C.

In Ausgangsstoffen der Formel IX kommt als abspaltbarer Rest Y beispielsweise eine reakionsfähig veresterte Hydroxygruppe, insbesondere Halogen, wie Chlor oder Brom in Betracht. Verbindungen der Formel IX sind bekannt und auch käuflich zu erwerben.

Verbindungen der Formel X lassen sich beispielsweise durch Ringschluss aus entsprechenden Verbindungen der Formel XII

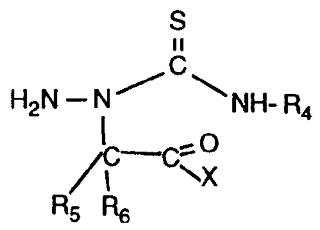

(XII)

worin $R_4$,$R_5$ und $R_6$ die oben angegebenen Bedeutungen haben, erhalten.

Die Kondensation erfolgt in Gegenwart eines basischen Kondensationsmittels, wie einer tertiären organischen Base, wie eines Triniederalkylamins, von Hünig'scher Base oder einer organischen Stickstoffbase, wie Chinolin, Pyridin oder insbesondere Dimethylaminopyridin, vorteilhafterweise in einem Temperaturberei von 20°C und Rückflusstemperatur des inerten Lösungsmittels, insbesondere zwischen 50°C und Rückflusstemperatur des verwendeten inerten Lösungsmittels. Als inertes Lösungsmittel verwendet man vorteilhafterweise ein Niederalkanol, insbesondere Ethanol.

Verbindungen der Formel XII lassen sich aus entsprechenden Hydrazino-essigsäureestern, insbesondere dem Ethylester durch Kondensation mit einem Isothiocyanat der Formel XIII

$R_4$-CNS (XIII)

worin $R_4$ die oben angegebenen Bedeutungen hat, erhalten.

Die Kondensation erfolgt in analoger Weise wie die der Verbindungen der X mit Verbindungen der Formel XI. Die nach den Verfahrensvarianten a)-d) erfindungsgemäss erhältlichen als Isomerengemisch vorliegenden Verbindungen der Formel I können gewünschtenfalls in die einzelnen Isomeren aufgetrennt werden und/oder erfindungsgemäss erhältliche freie Verbindungen in ein Salz oder ein erfindungsgemäss erhältliches Salz in eine freie Verbindung oder in ein anderes Salz überführt werden.

Verfahrensgemäss erhältliche Verbindungen können in üblicher Weise in andere Verbindungen der Formel I überführt werden.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw freien Verbindungen zu verstehen.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Müssigkeiten, wie fetten Ölen, Paraffinöl oder flüssigen Polyethylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykol oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Erfindung betrifft ebenfalls die Verwendung der Verbindungen der Formel I, vorzugsweise in Form von pharmazeutischen Präparaten. Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 5 mg bis etwa 1000 mg, insbesondere von etwa 10 mg bis etwa 200 mg zu veranschlagen. Diese kann auf auf einmal gegeben oder auf mehrere, z.B. 2 bis 4 Einzeldosen verteilt werden. Pharmazeutische Präparate in Dosiseinheitsform enthalten somit von etwa 5 mg bis etwa 250 mg, insbesondere von etwa 10 mg bis etwa 50 mg.

Die nachfolgen Beispiele dienen zur Illustration der Erfindung; Temperaturen sind in Celsiusgraden, Drucke in mbar angegeben.

Beispiel 1:

Zu einem Gemisch von 6 g 3-Allyl-thiazolidin-2,4-dion-2-(4-methyl-3-thiosemicarbazon) und 7,5 ml Triethylamin in 200 ml absolutem Methlenchlorid werden unter Rühren bei -55° bis -60° innerhalb von 5 Minuten 2,5 ml Oxalylchlorid zugetropft und anschliessend 30 Minuten bei derselben Temperatur weitergerührt. Das Gemisch wird auf 0° aufwärmen gelassen und 15 Minuten bei derselben Temperatur gerührt. Die orange Lösung wird zweimal mit Eiswasser und einmal mit verdünnter Natriumhydrogencarbonat-Lösung extrahiert und über Natriumsulfat getrocknet. Aus dem nach dem Abziehen des Lösungsmittels verbleibenden Öles werden aus 50 ml Ethanol 4,6 g leicht oranger Kristalle mit Schmelzpunkt 126-127° erhalten. Umkristallisation aus Ethanol mit einigen Tropfen Methylenchlorid ergab die analysenreine Verbindung 1-(3-Allyl-4-oxo-thiazolidin-2-yliden)-1-amino-3-methylimidazolidin-2-thion-4,5-dion. Schmelzpunkt 128° $^1$H-NMR : 3.45 (s, 3H), 3.95 (s, 2H), 4.5 (d, 2H), 5.25-5.4 (m, 2H), 5.9 (m, 1H).

Beispiel 2:

In analoger Weise zu Beispiel 1 werden aus 6,2 g 3-Methallyl-5,5-dimethylthiazolidin-2,4-dion-2-(4-allyl-3-thiosemicarbazon) und 2,7 g Oxalylchlorid nach Umkristallisation aus Ethanol 1-(3-Methallyl-5,5-dimethyl-4-oxo-thiazolidin-2-yliden)-1-amino-3-allyl-imidazolidin-2-thion-4,5-dion erhalten. Schmelzpunkt: 144-145°; $^1$H-NMR: 1.65 (s, 6H), 1.8 (s, 3H), 4.4 (s, 2H), 4.55 (d, 2H), 4.85 (s, 1H), 4.95 (s, 1H), 5.3 (m, 2H), 5.75-5.9 (m, 1H).

Beispiel 3:

In analoger Weise zu Beispiel 1 werden aus 2,5 g 3-Methallyl-5,5-dimethylthiazolidin-2,4-dion-2-(4-methyl-3-thiosemicarbazon) und 1,1 g Oxalylchlorid nach Umkristallisation aus Ethanol 1-(3-Methallyl-5,5-dimethyl-4-oxo-thiazolidin-2-yliden)-1-amino-3-methyl-imidazolidin-2-thion-4,5-dion. Schmelzpunkt: 155-156°;
$^1$H-NMR: 1.7 (s, 6H), 1.8 (s, 3H), 3.45 (s, 3H), 4.45 (s, 2H), 4.85 (s, 1H), 4.95 (s, 1H).

Beispiel 4:

In analoger Weise zu Beispiel 1 werden aus 4,9 g 3-Methyl-thiazolidin-2,4-dion-2-(4-allyl-3-thiosemicarbazon) und 1,9 g Oxalylchlorid nach Umkristallisation aus Ethanol 1-(3-Methyl-4-oxo-thiazolidin-2-yliden)-1-amino-3-allyl-imidazolidin-2-thion-4,5-dion-erhalten. Schmelzpunkt: 152-154°;
$^1$H-NMR: 3.35 (s, 3H), 3.9 (s, 2H), 4.5 (d, 2H), 5.25-5.35 (m, 2H), 5.8-5.95 (m, 1H).

Beispiel 5:

In analoger Weise zu Beispiel 1 werden aus 2 g 3-Allyl-thiazolidin-2,4-dion-2-(4-allyl-3-thiosemicarbazon) und 0,75 ml Oxalylchlorid nach Umkristallisation aus Ethanol 1-(3-Allyl-4-oxo-thiazolidin-2-yliden)-1-amino-3-allyl-imidazolidin-2-thion-4,5-dion erhalten. Schmelzpunkt: 138°;
$^1$H-NMR: 3.95 (s, 2H), 4.45 (d, 2H), 4.6 (d, 2H), 5.25-5.4 (m, 4H), 5.8-6.0 (m, 2H).

Beispiel 6:

In analoger Weise zu Beispiel 1 werden aus 2 g 3-Propyl-thiazolidin-2,4-dion-2-(4-methyl-3-thiosemicarbazon) und 1,14 g Oxalylchlorid nach Umkristallisation aus Ethanol 1-(3-Propyl-4-oxo-thiazolidin-2-yliden)-1-amino-3-methyl-imidazolidin-2-thion-4,5-dion erhalten. Schmelzpunkt: 112-113°;
$^1$H-NMR: 0.95 (t, 3H), 1.8 (dxq, 2H), 3.45 (s, 3H), 3.85 (t, 2H), 3.9 (s, 2H).

Beispiel 7:

In analoger Weise zu Beispiel 1 werden aus 1 g 3-Propinyl-thiazolidin-2,4-dion-2-(4-methyl-3-thiosemicarbazon) und 0,55 g Oxalylchlorid nach Umkristallisation aus Ethanol 1-(3-Propinyl-4-oxo-thiazolidin-2-yliden)-1-amino-3-methyl-imidazolidin-2-thion-4,5-dion erhalten. Schmelzpunkt: 173-174°;
$^1$H-NMR: 2.3 (br. s, 1H), 3.4 (s, 3H), 3.95 (s, 2H), 4.6 (br, s 2H).

Beispiel 8:

Zu einer Mischung von 4,9 g 3-Allyl-thiazolidin-2,4-dion-2-(4-methyl-3-thio-semicarbazon) und 6,2 ml Triethylamin in 100 ml absolutem Methylenchlorid werden unter Rühren bei -60° 2,7 ml 2-Bromisobuttersäurebromid während 5 Minuten zugetropft und 30 Minuten bei derselben Temperatur gerührt. Die Suspension wird auf Raumtemperatur aufwärmen gelassen und das Gemisch zwischen Wasser und Methylenchlorid verteilt. Die wassrige Phase wird ein zweites Mal extrahiert und die vereinigten organischen Phasen über Natriumsulfat getrocknet und eingeengt. Aus kaltem Ethanol werden farblose Kristalle eines Nebenproduktes mit Azin-Struktur abgetrennt und die Mutterlauge wird eingeengt, in 30 ml Chloroform aufgenommen, mit 5 ml Triethylamin versetzt und 2 Stunden am Rückfluss gekocht. Nach dem Abkühlen auf Raumtemperatur wird das Gemisch zwischen Wasser und Chloroform verteilt und die organische Phase nach dem Trocknen über Magnesiumsulfat eingeengt. Aus Ethanol werden Kristalle erhalten, die nach Umkristallisation aus Methylenchlorid/Diethylether die reine Verbindung 1-(3-Allyl-4-oxo-thiazolidin-2-yliden)-1-amino-3-methyl-5,5-dimethylimidazolidin-2-thion-4-on ergeben. Schmelzpunkt: 142°;
$^1$H-NMR: 3.3 (s, 3H), 3.9 (s, 2H), 4.5 (d, 2H), 5.25-5.4 (m, 2H), 5.9 (m, 1H).

Beispiel 9:

a) In 15 ml Ethanol und 0,4 g Triethylamin werden 1 g Verbindung der Formel VIII worin $R_1$ Allyl und Y Methyl bedeutet und 0,4 g Sarcosin für 3,5 Stunden am Rückfluss gekocht. Das Lösungsmittel wird abgezogen, der Rückstand in Methylenchlorid aufgenommen und mit 0.1 N Salzsäure und Wasser gewaschen. Nach dem Trocknen über Magnesiumsulfat und Abziehen des Lösungsmittels verbleibt ein gelbliches Oel, wovon aus Ethanol/Methylenchlorid die reine Verbindung 1-(3-Allyl-4-oxo-thiazolidin-2-yliden)-1-amino-3-methyl-imidazolidin-2-thion-5-on kristallisiert erhalten wird. Schmelzpunkt: 131-133°;
$^1$H-NMR: 3.3 (s, 3H), 3.9 (s, 2H), 4.5 (d, 2H), 5.25-5.4 (m, 2H), 5.9 (m, 1H).

b) Das Zwischenprodukt der Formel VIII, worin $R_1$ Allyl und Y Methyl bedeuten, wird wie folgt erhalten:
In 100 ml Tetrahydrofuran werden 10 g 3-Allyl-2,4-thiazolidindion-2-hydrazon gelöst und mit 8,2 ml Triethylamin und 3,6 ml Schwefelkohlenstoff versetzt und 3 Stunden bei Raumtemperatur gerührt. Die Mischung wird auf -5° gekühlt und anschliessend 4 ml Methyljodid zugetropft und die Mischung auf Raumtemperatur aufwärmen gelassen und 2 Stunden gerührt. Darauf wird das Gemisch mit Wasser versetzt und das ausgefallene Produkt filtriert. Der Festkörper wird in Methylenchlorid gelöst und über Magnesiumsulfat getrocknet. Nach dem Abziehen von 2/3 des Gesamtvolumens des Lösungsmittels wird das auskristallisierte Produkt abfiltriert und an der Luft getrocknet. Aus der Mutterlauge kann nach Chromatographie an -Kieselgel mit Methylenchlorid nochmals Produkt erhalten werden. Schmelzpunkt: 148-150°;
$^1$H-NMR: 2.6 (s, 3H), 4.0 (s, 2H), 4.4 (d, 2H), 5.25-5.45 (m, 2H), 5.9 (m, 1H), 8.3 (br. s, 1H).

Beispiel 10:

In analoger Weise zu Beispiel 9 werden 2.0g Verbindung der Formel V, worin $R_1$ Allyl, $R_2$, $R_3$ und Y Methyl bedeuten und 1.23g Sarcosinethylester in 25 ml Ethanol mit einer Spatelspitze Dimethylaminopyridin für 6 Stunden am Rückfluss gekocht. Das Gemisch wird auf 0°C abgekühlt und über Nacht kristallisiert bei dieser Temperatur das Produkt aus, welches filtriert und mit wenig Ether gewaschen und anschliessend getrocknet wird. Man erhält, gegebenenfalls nach Umkristallisation aus Methylenchlorid/Ether, die reine Verbindung 1-(3-Allyl-5,5-dimethyl-4-oxo-thiazolidin-2-yliden)-1-amino-3-methyl-imidazolidin-2-thion-5-on. Schmelzpunkt: 132-133°C.

Beispiel 11:

In analoger Weise zu Beispiel 9 werden 3,5g Verbindung der Formel V, worin $R_1$ Allyl und Y Methyl bedeuten und 1.23g Alanin-methylester-hydrochlorid in 30 ml Ethanol mit 2.1 ml Triethylamin und einer Spatelspitze Dimethylaminopyridin für 8 Stunden am Rückfluss gekocht. Das Gemisch wird eingeengt und der Rückstand in Methylenchlorid aufgenommen. Man wäscht mit verdünnter Salzsäurelösung und danach mit verdünnter Natriumbicarbonatlösung, trocknet über Natriumsulfat und zieht das Lösungsmittel am Rotationsverdampfer ab. Nach Chromatographie an Kieselgel wird neben ofenkettigem Thiosemicarbazon als Nebenprodukt das cyclisierte Produkt erhalten.

Die ölige Verbindung erstarrt am Hochvakuum zu einem glasigen Schaum von 1-(3-Allyl-4-oxo-thiazolidin-2-yliden)-1-amino-4-methyl-imidazolidin-2-thion-5-on. $^1$H-NMR: 1.55 (d,3H), 3.9 (s,2H), 4.25 (q,1H), 4.5 (d,2H), 5.3 (m,2H), 5.8 (m,1H), 7.85 (br.s, 1H).

Beispiel 12:

Zu 12g 1-(3-Methyl-4-oxo-2-thioxo-imidazolidin-1-yl)-3-allyl-thioharnstoff und 9g wasserfreiem Natriumacetat in 200 ml Ethanol werden 7.5g Bromessigsäure zugegeben und das Gemisch wird bei 60°C für 6 Stunden gerührt. Das Lösungsmittel wird abgezogen und der Rückstand zwischen Wasser/Methylenchlorid verteilt. Die organische Phase wird mit Natriumsulfat getrocknet und nach Filtration mit 150 ml Ethanol versetzt. Das Methylenchlorid wird am Rotationsverdampfer abgzogen, wobei das Produkt kristallisiert. Das Gemisch wird auf 0°C abgekühlt und das Produkt abfiltriert, mit Ether gewaschen und getrocknet. Man erhält, gegebenenfalls nach Umkristallisation aus Methylenchlorid/Ether, die reine Verbindung 1-(3-Allyl-4-oxo-thiazolidin-2-yliden)-1-amino-3-methyl-imidazolidin-2-thion-4-on mit einem Schmelzpunkt von 136-137°C.

Beispiel 13:

Zu 4.8g 1-(3-Methyl-4-oxo-2-thioxo-imidazolidin-1-yl)-3-allyl-thioharnstoff und 4g wasserfreiem Natriumacetat in 50 ml Ethanol werden 3.8g 2-Brom-isobuttersäure zugegeben und das Gemisch wird bei 50°C für 15 Stunden gerührt. Das Lösungsmittel wird abgezogen und der Rückstand zwischen Wasser/Methylenchlorid verteilt. Die organische Phase wird mit Natriumsulfat getrocknet und eingeengt. Das Gemisch wird an Kieselgel mit Methylenchlorid chromatographiert und das Produkt anschliessend aus Methylenchlorid/Ether kristallisiert. man erhält die reine Verbindung 1-(3-Allyl-5,5-dimethyl-4-oxo-thiazolidin-2-yliden)-1-amino-3-methyl-imidazolidin-2-thion-4-on, Schmelzpunkt 121-122°C.

Beispiel 14:

Tabletten, enthaltend je 10 mg 1-(3-Allyl-4-oxo-thiazolidin-2-yliden)-1-amino-3-methyl-imidazolidin-2-thion-4,5-dion oder ein Salz davon, können wie folgt hergestellt werden:

Zusammensetzung (10000 Tabletten)

| | |
|---|---|
| Wirkstoff | 100,0 g |
| Lactose | 450,0 g |
| Kartoffelstärke | 350,0 g |
| Gelatine | 10,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 50,0 mg Wirkstoffgehalt, die gewünschtenfalls mit Teilkerben zur feineren Anpassung der Dosierung versehen sein können.

Beispiel 15:

Gelatinesteckkapseln, enthaltend 20 mg Wirkstoff, 1-(3-Allyl-4-oxo-thiazolidin-2-yliden)-1-amino-3-methyl-imidazolidin-2-thion-4,5-dion oder ein Salz davon, können z.B. folgendermassen hergestellt werden:

Zusammensetzung (für 1000 Kapseln)

| | |
|---|---|
| Wirkstoff | 20,0 g |
| Lactose | 240,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das

Magnesiumstearat durch ein Sieb mit einer Maschenweite von 0,8 mm hinzugesiebt. Nach 3- minütigem weiteren Mischen werden je 300 mg der erhaltenen Formulierung in Gelatinesteckkapseln der Grösse 0 abgefüllt.

Beispiel 16:

Gelatinesteckkapseln, enthaltend 100 mg Wirkstoff, z.B. 1-(3-Allyl-4-oxo-thiazolidin-2-yliden)-1-amino-3-methyl-imidazolidin-2-thion-4,5-dion oder ein Salz davon, können z.B. folgendermassen hergestellt werden:

Zusammensetzung (für 1000 Kapseln)

| | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 17:

Lacktabletten, enthaltend je 50 mg 1-(3-Allyl-4-oxo-thiazolidin-2-yliden)-1-amino-3-methyl-imidazolidin-2-thion-4,5-dion oder ein Salz davon, können wie folgt hergestellt werden:

Zusammensetzung (für 1000 Lacktabletten)

| | |
|---|---|
| Wirkstoff | 50,0 g |
| Lactose | 100,0 g |
| Maisstärke | 70,0 g |
| Talk | 10,0 g |
| Calciumstearat | 2,0 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 240 mg) verpresst und diese mit einer Lösung der Hydroxypropylmethylcellulose und des Schellacks in Methylenchlorid lackiert; Endgewicht der Lacktablette: 283 mg.

Beispiel 18:

Eine 0,2%-ige Injektions- oder Infusionslösung von 1-(3-Allyl-4-oxo-thiazolidin-2-yliden)-1-amino-3-methyl-imidazolidin-2-thion-4,5-dion oder ein Salz davon, kann beispielsweise folgendermassen hergestellt werden:

Zusammensetzung (für 1000 Ampullen)

| | |
|---|---|
| Wirkstoff | 5,0 g |
| Natriumchlorid | 22,5 g |
| Phosphatpuffer pH= 7,4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 19:

1%-ige Salbe (O/W-Emulsion), als Wirkstoff z.B 1-(3-Allyl-4-oxo-thiazolidin-2-yliden)-1-amino-3-methyl-imidazolidin-2-thion-4,5-dion oder ein Salz davon, enthaltend, mit folgender Zusammensetzung:

| | |
|---|---|
| Wirkstoff | 1,0 g |
| Cetylalkohol | 3,0 g |
| Glycerin | 6,0 g |
| Methylparaben | 0, 18 g |
| Propylparaben | 0,05 g |
| Arlacel 60 | 0,6 g |
| Tween 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

Beispiel 20:

1%-iges Gel, als Wirkstoff z.B. 1-(3-Allyl-4-oxo-thiazolidin-2-yliden)-1-amino-3-methyl-imidazolidin-2-thion-4,5-dion oder ein Salz davon, enthaltend, mit folgender Zusammensetzung:

| | |
|---|---|
| Wirkstoff | 1,0 g |
| Carbopol 934 P | 1,0 g |
| Glycerin | 3,0 g |
| Isopropanol | 25,0 g |
| Softigen® 767 | 0,2 g |
| Wasser entmin. q.s. ad | 100,0 g |

Beispiel 21:

In analoger Weise wie in den vorstehenden Beispielen 14 bis 20 beschrieben kann man auch pharmazeutische Präparate, enthaltend eine andere Verbindung der Formel I oder ein pharmazeutisch verwendbares Salz davon herstellen.

## Patentansprüche

1. Eine Verbindung der Formel I

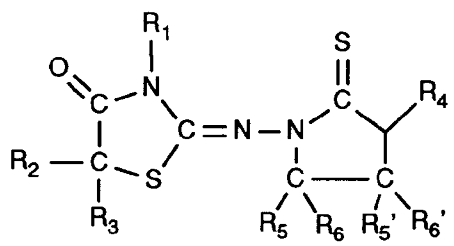

(I)

worin $R_1$ und $R_4$ Niederalkyl, Niederalk-2-en-1-yl oder auch Niederalk-2-in-1-yl bedeuten, $R_2$ und $R_3$ unabhängig voneinander Wasserstoff oder Niederalkyl bedeuten oder gemeinsam für Niederalkyliden stehen, und $R_5$ und $R_6$ jeweils für Wasserstoff oder Niederalkyl stehen oder gemeinsam Oxo darstellen, und $R_5$' und $R_6$' die gleiche Bedeutung von $R_5$ und $R_5$ haben, mit der Massgabe, dass mindestens einer der Substituentenpaare $R_5$ und $R_6$ oder $R_5$' und $R_5$' gemeinsam für Oxo stehen und Salze davon.

2. Eine Verbindung gemäss Anspruch 1 der Formel I, worin $R_1$ $C_3$-$C_5$-Alk-2-en-1-yl oder $C_3$-$C_5$-Alk-2-in-1-yl und $R_4$ $C_1$-$C_4$-Alkyl, $C_3$-$C_5$-Alk-2-en-1-yl oder $C_3$-$C_5$-Alk-2-in-1-yl bedeutet, $R_2$ und $R_3$ unabhängig voneinander Wasserstoff oder $C_1$-$C_4$-Alkyl bedeuten oder gemeinsam $C_1$-$C_4$-Alkyliden darstellen und $R_5$ und

$R_6$ bzw. $R_5$' und $R_6$' jeweils unabhängig voneinander Wasserstoff oder $C_1$-$C_4$-Alkyl oder gemeinsam Oxo darstellen, mit der Massgabe, dass mindestens einer der Substituentenpaare $R_5$ und $R_6$ oder $R_5$' und $R_6$' gemeinsam für Oxo stehen, und Salze davon.

3. Eine Verbindung gemäss Anspruch 1 der Formel I, worin der Rest $R_1$ $C_3$-$C_5$-Alk-2-en-1-yl, wie Allyl oder Methallyl oder $C_3$-$C_5$-Alk-2-in-1-yl, wie Prop-2-in-2-yl, und $R_4$ $C_1$-$C_4$-Alkyl, wie Methyl, oder $C_3$-$C_5$-Alk-2-en-1-yl, wie Allyl oder Methallyl, bedeutet, $R_2$ und $R_3$ beide Wasserstoff oder gleiche $C_1$-$C_4$-Alkylgruppen, wie Methyl, bedeuten oder gemeinsam $C_1$-$C_4$-Alkyliden, wie Methylen darstellen und $R_5$ und $R_6$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_5$' und $R_5$' gemeinsam für Oxo stehen, und pharmazeutisch verwendbare Salze davon.

4. Eine Verbindung gemäss Anspruch 1 der Formel I, worin $R_1$ Allyl oder Methallyl, $R_2$ und $R_3$ beide Wasserstoff oder Methyl bedeuten, $R_4$ für Methyl, Allyl oder Methallyl stehen und $R_5$ und $R_5$ bzw. $R_5$' und $R_6$' jeweils Wasserstoff oder gemeinsam Oxo darstellen, mit der Massgabe, dass mindestens einer der Substituentenpaare $R_5$ und $R_6$ oder $R_5$' und $R_5$' gemeinsam für Oxo stehen, und pharmazeutisch verwendbare Salze davon.

5. 1-(3-Allyl-4-oxo-thiazolidin-2-yliden)-1-amino-3-methyl-imidazolidin-2-thion-4,5-dion oder ein pharmazeutisch verwendbares Salz davon.

6. 1-(3-Methylallyl-5,5-dimethyl-4-oxo-thiazolidin-2-yliden)-1-amino-3-allyl-imidazolidin-2-thion-4,5-dion oder ein pharmazeutisch verwendbares Salz davon.

7. 1-(3-Methylallyl-5,5-dimethyl-4-oxo-thiazolidin-2-yliden)-1-amino-3-methyl-imidazolidin-2-thion-4,5-di on oder ein pharmazeutisch verwendbares Salz davon.

8. 1-(3-Methyl-4-oxo-thiazolidin-2-yliden)-1-amino-3-allyl-imidazolidin-2-thion-4,5-dion oder ein pharmazeutisch verwendbares Salz davon.

9. 1-(3-Allyl-4-oxo-thiazolidin-2-yliden)-1-amino-3-allyl-imidazolidin-2-thion-4,5-dion oder ein pharmazeutisch verwendbares Salz davon.

10. 1-(3-Propyl-4-oxo-thiazolidin-2-yliden)-1-amino-3-methyl-imidazolidin-2-thion-4,5-dion oder ein pharmazeutisch verwendbares Salz davon.

11. 1-(3-Propinyl-4-oxo-thiazolidin-2-yliden)-1-amino-3-methyl-imidazolidin-2-thion-4,5-dion oder ein pharmazeutisch verwendbares Salz davon.

12. 1-(3-Allyl-4-oxo-thiazolidin-2-yliden)-1-amino-3-methyl-5,5-dimethyl-imidazolidin-2-thion-4-on oder ein pharmazeutisch verwendbares Salz davon.

13. 1-(3-Allyl-4-oxo-thiazolidin-2-yliden)-1-amino-3-methyl-imidazolidin-2-thion-5-on oder ein pharmazeutisch verwendbares Salz davon.

14. 1-(3-Allyl-5,5-dimethyl-4-oxo-thiazolidin-2-yliden)-1-amino-3-methyl-imidazolidin-2-thion-5-on oder ein pharmazeutisch verwendbares Salz davon.

15. 1-(3-Allyl-4-oxo-thiazolidin-2-yliden)-1-amino-4-methyl-imid azolidin-2-thion-5-on oder ein pharmazeutisch verwendbares Salz davon.

16. 1-(3-Allyl-4-oxo-thiazolidin-2-yliden)-1-amino-3-methyl-imidazolidin-2-thion-4-on oder ein pharmazeutisch verwendbares Salz davon.

17. 1-(3-Allyl-5,5-dimethyl-4-oxo-thiazolidin-2-yliden)-1-amino-3 ethyl-imidazolidin-2-thion-4-on oder ein pharmazeutisch verwendbares Salz davon.

18. Verbindungen gemäss einem der Ansprüche 1 bis 17 zur Anwendung in einem Verfahren zur therapeutischen Behandlung des menschlichen oder tierischen Körpers.

19. Pharmazeutische Präparate, neben üblichen pharmazeutischen Hilfsstoffen als pharmazeutischen Wirkstoff enthaltend eine Verbindung gemäss einem der Ansprüche 1 bis 17 in freier Form oder in pharmazeutisch verwendbarer Salzform.

**20.** Die Verbindungen der allgemeinen Formel I gemäss Anspruch 1 als wirksames Mittel zur Behandlung von Erkrankungen des rheumatoiden Formenkreises.

**21.** Die Verwendung von Verbindungen der allgemeinen Formel I gemäss Anspruch 1 und übliche Trägerstoffe zur Herstellung von pharmazeutischen Präparaten.

**22.** Die Verwendung der neuen Verbindungen der Formel I zur Behandlung von Erkrankungen des rheumatoiden Formenkreises.

**23.** Verfahren zur Herstellung neuer Verbindungen der Formel I gemäss Anspruch 1, dadurch gekennzeichnet, dass man

a) eine Verbindung der Formel II

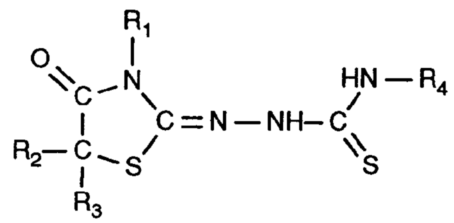

(II)

mit einer entsprechenden Verbindung der Formel III

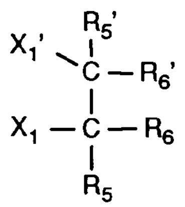

(III)

worin $X_1$ und $X_1'$ jeweils ein reaktionsfähiges verestertes Hydroxy bedeuten und $R_5$, $R_6$, $R_5'$ und $R_6'$ die oben angegebenen Bedeutungen haben, in Gegenwart eines basischen Kondensationsmittel kondensiert oder

b) in einer Verbindung der Formel IV

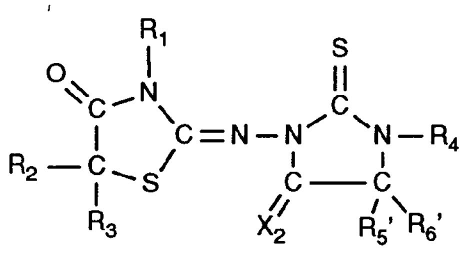

(IV)

worin $X_2$ eine funktionell abgewandelte Oxogruppe darstellt und $R_1$, $R_2$, $R_3$, $R_4$, $R_5'$ und $R_6'$ die angegebenen Bedeutungen haben, oder die entsprechenden Salzen davon die Gruppe $X_2$ in Oxo überführt oder

c) eine Verbindung der Formel V

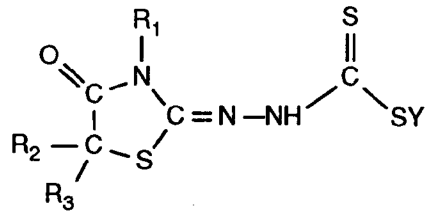

(V)

worin $R_1$, $R_2$, $R_3$ die oben angegebenen Bedeutungen haben und Y Niederalkyl bedeutet, mit einer Verbindung der Formeln VI

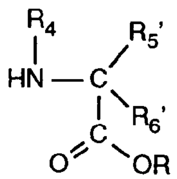

(VI)

worin $R_4$, $R_5$' und $R_6$' die oben angegebenen Bedeutungen haben und R Wasserstoff oder Niederalkyl bedeutet, umsetzt oder
d) eine Verbindung der Formel VIII

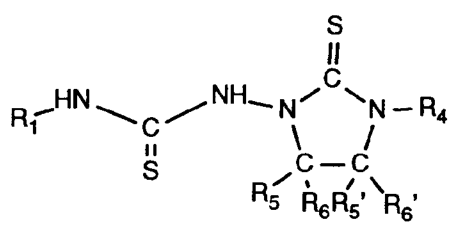

(VIII)

worin $R_1$, $R_4$, $R_5$, $R_6$, $R_5$' und $R_5$' die oben angegebenen Bedeutungen haben, mit einer Verbindung der Formel IX

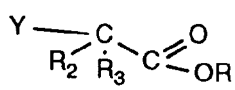

(IX)

worin $R_2$ und $R_3$ die oben angegebenen Bedeutungen haben, Y ein reaktionsfähiges verestertes Hydroxy als apspalbaren Rest und R Wasserstoff oder Niederalkyl bedeuten, umsetzt und gewünschtenfalls ein erfindungsgemäss erhältliches Isomerengemisch in die individuellen Isomeren auftrennt und das gewünschte Isomere isoliert und/oder eine erfindungsgemäss erhältliche freie Verbindung in ein Salz oder ein erfindungsgemäss erhältliches Salz in die freie Verbindung oder in ein anderes Salz überführt.

Europäisches Patentamt

# EUROPÄISCHER RECHERCHENBERICHT

Nummer der Anmeldung

EP 92 81 0972

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 432 740 (HOECHST A.G.)<br>* Beispiel 4 * | 1-22 | C07D417/12<br>A61K31/425 |
| A | CHEMICAL ABSTRACTS, vol. 102, no. 11, 18. März 1985, Columbus, Ohio, US; abstract no. 89678x, M.G. VIGORITA ET AL.<br>* Zusammenfassung *<br>& FARMACO, ED. SCI. Bd. 39, Nr. 12, 1984, Seiten 1008 - 23 | 1-22 | |
| A | EP-A-0 069 784 (MITSUI TOATSU KAGAKU KABUSHIKI)<br>* Tabelle 1 * | 1-22 | |
| A | EP-A-0 069 154 (MITSUI TOATSU KAGAKU KABUSHIKI)<br>* Tabelle 1 * | 1-22 | |

| RECHERCHIERTE SACHGEBIETE (Int. Cl.5 ) |
|---|
| C07D<br>A61K |

Der vorliegende Recherchenbericht wurde für alle Patentansprüche erstellt

| Recherchenort | Abschlußdatum der Recherche | Prüfer |
|---|---|---|
| BERLIN | 05 FEBRUAR 1993 | FRELON D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)